# EUROPEAN PATENT SPECIFICATION

(11) **EP 2 627 195 B1**
(45) Date of publication and mention of the grant of the patent: **20.08.2014**
(21) Application number: 11773225.5
(22) Date of filing: 13.10.2011
(51) Int. Cl.: A23L 1/015, A23L 1/29, A23L 1/30, A61K 31/12

(54) **CURCUMINOID SOLID DISPERSION FORMULATION**
FESTE CURCUMMINOIDE DISPERSIONSFORMULIERUNG
FORMULATION CONTENANT UNE DISPERSION SOLIDE COMPRENANT UN OU PLUSIEURS CURCUMINOÏDES

(30) Priority: 14.10.2010 US 393206 P
(43) Date of publication of application: 21.08.2013
(73) Proprietor: Abbott GmbH & Co. KG, 65205 Wiesbaden (DE); Abbott Laboratories, Abbott Park, IL 60064 (US)
(72) Inventor: BREITENBACH, Jörg, 68199 Mannheim (DE); KESSLER, Thomas K., 67105 Schifferstadt (DE); SCHNEIDER, Katrin, 68163 Mannheim (DE); DAS, Tapas, Singapore 239192 (SG); SATHYA, Shreeram, Singapore 117687 (SG); CHUAH, Ai mey, Singapore 680416 (SG); PATEL, Gaurav C., Gahanna, Ohio 43230 (US)
(74) Representative: Ricker, Mathias
(86) International application number: PCT/EP2011/067901
(87) International publication number: WO 2012/049253

(56) References cited:
- WO-A2-2007/103435
- US-A1- 2006 067 998
- DATABASE WPI Week 200936 Thomson Scientific, London, GB; AN 2009-F76679 XP002664564, & CN 101 361 713 A (UNIV SHANDONG) 11 February 2009 (2009-02-11)

## Description

### FIELD OF THE INVENTION

Curcumin is the principal curcuminoid of the popular Indian spice turmeric, which is a member of the ginger family (Zingiberaceae). Other naturally occurring curcuminoids are desmethoxycurcumin and bis-desmethoxycurcumin. Curcuminoids are polyphenols and are responsible for the yellow color of turmeric. Curcuminoids can exist in at least two tautomeric forms, keto and enol. The enol form is more energetically stable in the solid phase and in solution. Curcumin is practically insoluble in water at acidic and neutral pH; and rapidly decomposes at alkaline pH. Curcumin is stable under dry conditions and also relatively stable to heat.

Curcumin is a very powerful antioxidant. Its antioxidant effect has been reported to be eight times more powerful than that of vitamin E. A number of studies provide evidence for the therapeutic properties of naturally occurring curcuminoids and synthetic curcuminoid derivatives, in particular their anti-cancer activity (for example, Pisano et al., Mol Cancer. 2010, 3;9(1):137; Bisht et al., J Nanobiotechnology. 2007, 5:3). There are also reports on other pharmacological activities of curcumin including anti-microbial and anti-inflammatory effects (Begum et al., J Pharmacol Exp Ther. 2008, 326(1):196-208).

Although curcuminoids have been suggested for a variety of therapeutic and prophylactic applications, a major impediment in this development is the very low bioavailability of orally administered curcumin. For example, it has been reported that serum levels in humans after an oral dose of 2 g curcumin alone were either undetectable or very low. Reasons contributing to this effect are the low stability and poor absorption of curcumin in the digestive tract as well as its rapid metabolism, in particular in the liver, and rapid systerriic elimination. Thus, the serum curcumin levels sufficient to provoke the desired beneficial effect of this compound cannot be achieved by the mere consumption of turmeric with the food. Concomitant administration of piperine, an inhibitor of enzymes involved in drug metabolism, has been shown to increase the curcumin ab- ' sorption and thus the serum concentration of curcumin. However, it is a significant downside of this approach that the piperine induced inhibition of drug metabolism may lead to unwanted effects, in particular when other medications are taken. (Anand et al., Mol Pharm 2007 4(6):807-18; Shoba et al., Planta Med 1998 64(4):353-6)

Different formulations of curcumin have been developed to circumvent the poor aqueous solubility and/or oral bioavailability of curcumin. In 2007, a polymeric nanoparticle- ' encapsulated formulation of curcumin ("nanocurcumin") with less than 100 nm particle size has been reported by Bisht ef al. (J Nanobiotechnology. 2007, 5:3) to be readily dispersable in aqueous media and to show comparable efficacy compared to free curcumin in human cancer cell line models. A "lipidated" curcumin formulation has been described by Begum et al. (J Pharmacol Exp Ther. 2008, 326(1):196-208) to result in 11-fold higher plasma curcumin levels and 4-fold higher levels in brain compared with equal doses of curcumin powder or curcumin-piperine extracts after oral administration. In WO2010/010431 liquid and semisolid self emulsifying curcumin formulations based on a lipid carrier system of PEG fatty acid esters are described which showed improved bioavailability compared to an aqueous suspension of curcumin after oral administration to rats. Curcumin nanoparticles and curcumin bound to chitosan nanoparticles were described to provide improved oral bioavailability of curcumin in mice compared to curcumin orally administered in olive oil (WO 2010/013224). Paradkar et al. (Int J ' Pharm. 2004, 271(1-2):281-6) describe curcumin-PVP solid dispersions obtained by spray drying that showed increased dissolution rates compared to pure curcumin or physical curcumin mixtures. Curcumin formulations including a nanocrystal solid dispersion in hydroxypropyl cellulose, an amorphous solid dispersion in hydroxypropylmethyl cellulose and a nanoemulsion in a solvent mixture comprising PEG400 have been described by Onoue et al. (J Pharm Sci. 2010, 99(4):1871-81). Each of these three curcumin formulations has been reported to show improved solubility in water as well as higher plasma curcumin concentrations in rats after oral administration compared to pure crystalline curcumin.

US 2006/067998 A relates to a colloidal drug delivery system, wherein curcumin or a respective analogue is encapsulated in a vesicle-forming lipid and wherein the colloidal drug delivery system is present in a pharmaceutically acceptable carrier, e.g. saline.

WO 2007/103435 A relates to a curcuminoid composition having an enhanced bioavailability and comprising a curcuminoid, an antioxidant and a water-solubilizing, pharmaceutically acceptable carrier and further optionally a glucuronidation inhibitor.

CN 101361713 relates to a nanocrystalline preparation comprising curcumin and a surface active agent, wherein the surface active agent is first dissolved in water and then curcumin is dispersed within said solution.

Despite the various approaches described above there is still a tremendous need to provide curcumin formulations which allow optimum bioavailability of curcumin when administered orally.

### SUMMARY OF THE INVENTION

Thus, this invention provides a curcuminoid formulation, comprising a melt-processed solid dispersion product comprising
a) one or more curcuminoids,
b) a nutritionally acceptable thermoplastic polymer, and
c) a phosphatide.

The solid dispersion product comprises a matrix of the nutritionally acceptable thermoplastic polymer and phosphatide wherein the curcuminoid(s) are homogeneously distributed. Beside the solid dispersion product, the curcuminoid formulation may comprise one or more other ingredients, e.g., additives or other nutritionally desirable ingredients.

The invention further provides a method for producing the curcuminoid formulation described herein, comprising:
a) blending one or more curcuminoids, a nutritionally acceptable thermoplastic polymer, and a phosphatide;
b) heating the blend to obtain a homogeneous melt;
c) forcing the thus obtained melt through one or more nozzles;
d) allowing the melt to solidify to obtain a solid dispersion product.

The invention also provides a nutritional product fortified with a curcuminoid formulation as described herein as well as the curcuminoid formulation described herein for use in the treatment or prophylaxis of diseases and conditions such as cancer, conditions involving an inflammatory reaction, neurological disorders, cardiovascular disease, pulmonary disease, the formation of cholesterol gallstones, and parasitic infestation.

### BRIEF SUMMARY OF THE FIGURES

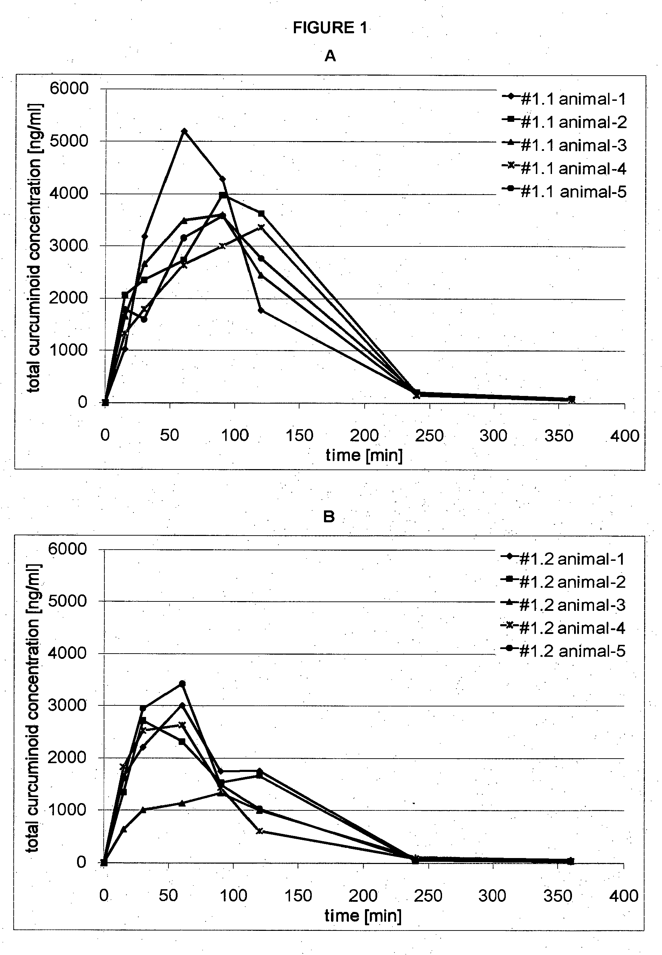

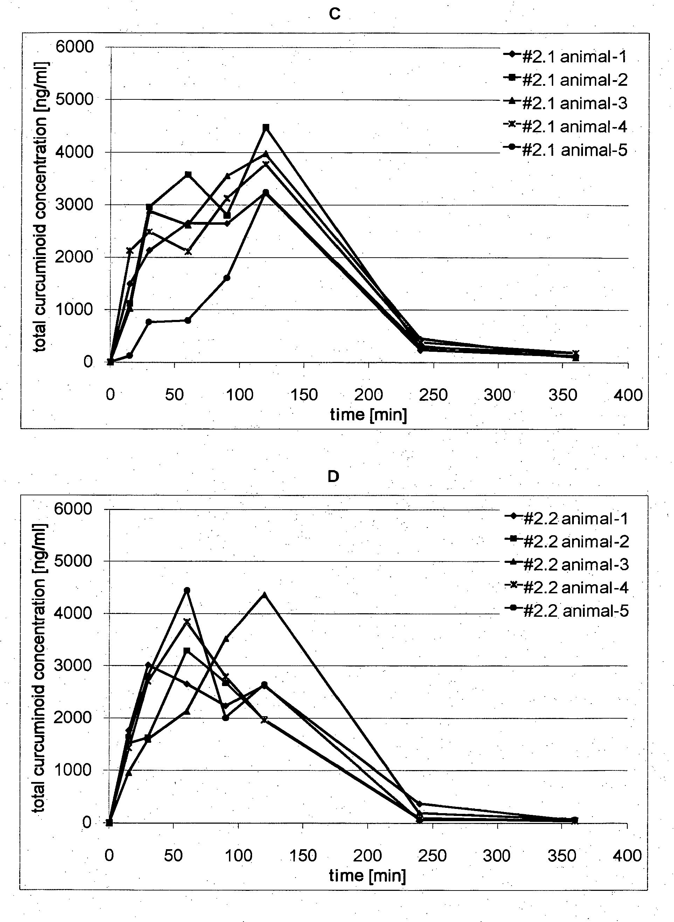

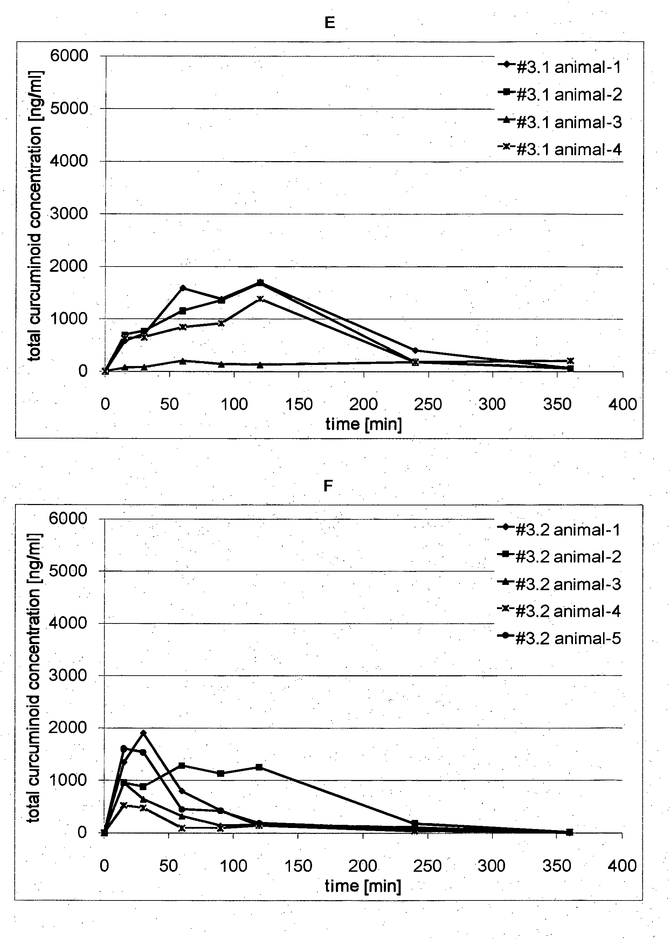

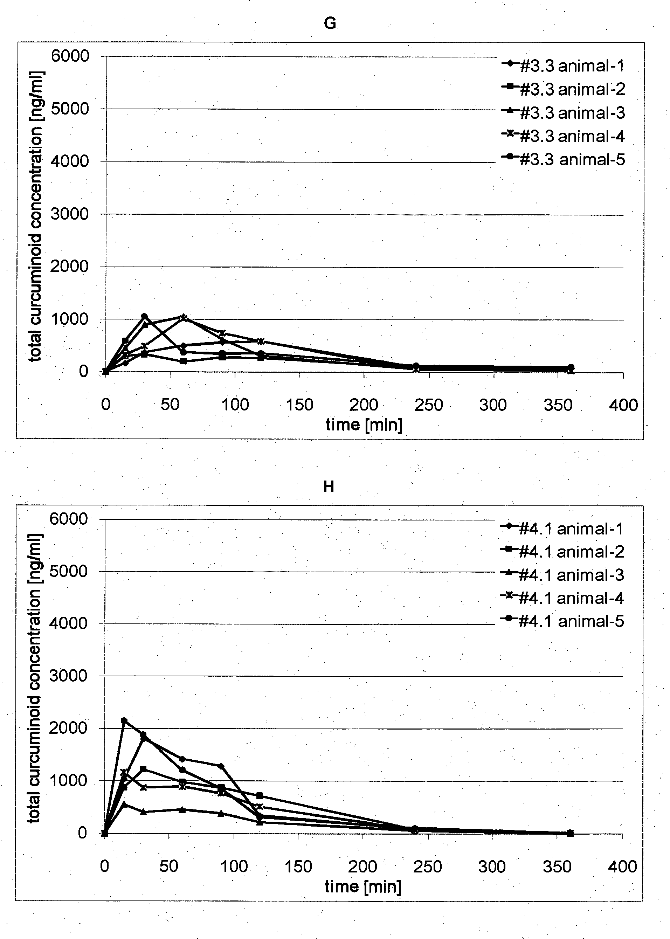

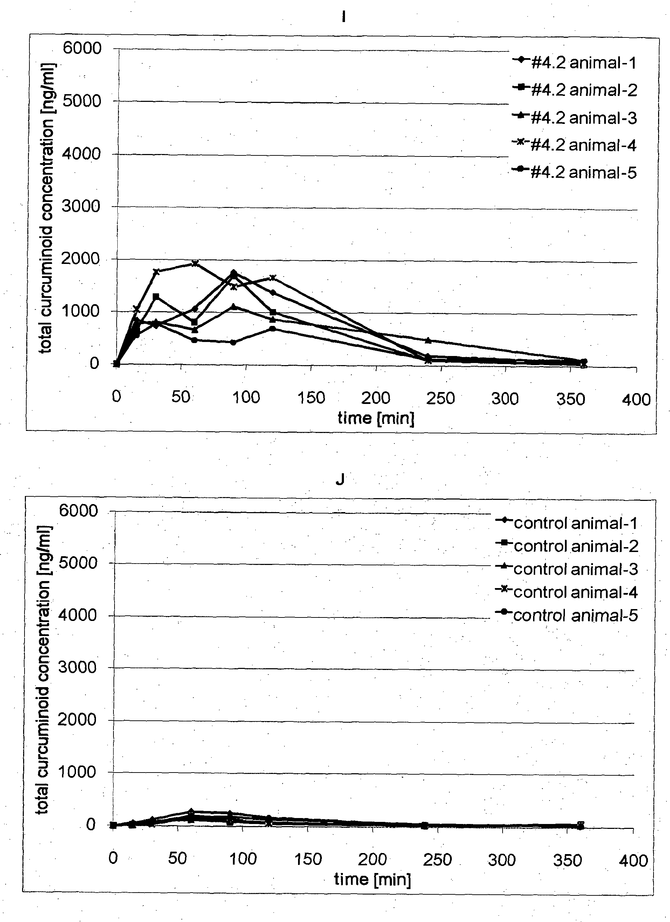

Figures 1 A-J show graphs illustrating the curcuminoid concentrations in rat plasma after oral administration of curcuminoids. 4 to 5 rats were orally administered with an amount of curcuminoid formulation #1.1, #1.2, #2.1, #2.2, #3.1, #3.2, #3.3, #4.1 or #4.2 comprising 20 mg curcuminoids (Figures 1 A-I). The control subjects were administered with 20 mg non-formulated crystalline curcuminoids (Figure 1 J).

### DETAILED DESCRIPTION OF THE INVENTION

The term "curcuminoid", as used herein, refers to curcumin and derivatives thereof and analogs thereof. These include natural and synthetic derivatives of curcumin, and any combination of more than one curcuminoid.

In particular, for the purposes herein, the term "curcuminoid" should be understood to encompass compounds having a 1,7-bis (4-hydroxyphenyl)-1,6-heptadiene-3,5-dione or 1,7-bis(4-hydroxyphenyl)hept-4-en-3-one skeleton wherein the phenyl groups independently may bear one or more alkoxy residues, especially one methoxy residue in 3-position.

Naturally occurring curcuminoids comprise curcumin (1,7-Bis-(4-hydroxy-3-methoxyphenyl)-hepta-1,6-diene-3,5-dione), desmethoxycurcumin (1-(4-Hydroxyphenyl)-7-(4-hydroxy-3-methoxyphenyl)-hepta-1,6-diene-3,5-dione) and bis-desmethoxycurcumin (1,7-Bis-(4-hydroxyphenyl)-hepta-1,6-diene-3,5-dione). These compounds can exist in at least two tautomeric forms, keto and enol having the structural formulae 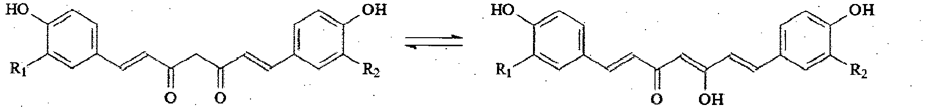 , wherein in curcumin R₁ and R₂ are methoxy, in desmethoxycurcumin R₁ is hydrogen and R₂ is methoxy, and in bis-desmethoxycurcumin R₁ and R₂ are hydrogen.

Analogs of curcumin include those derivatives disclosed in U.S. Patent 6,664,272 and Pisano et al., Mol Cancer. 2010, 3; 9(1):137, which are herein specifically incorporated by reference.

The term "nutritionally acceptable", as used herein, refers to a compound that does not cause acute toxicity when the curcumin formulation of the invention comprising it is ingested as a supplement for a nutritional product, or is administered orally in treatment or prophylaxis of diseases and conditions such as cancer, conditions involving an inflammatory reaction, neurological disorders, cardiovascular disease, pulmonary disease, the formation of cholesterol gallstones, and parasitic infestation. Expediently, all components of the curcumin formulation of the present invention are nutritionally acceptable.

The nutritionally acceptable thermoplastic polymer used in the solid dispersion product described herein is a polymer capable to act as a solid meltable solvent. It forms a matrix for dispersion, and in particular for dissolution, of the curcuminoid. Preferably, said polymer is at least partly soluble or swellable in aqueous media, expediently under the conditions of use, i.e. in particular under physiological conditions in the digestive tract. Most preferably, said polymer is a water-soluble polymer.

The nutritionally acceptable thermoplastic polymer may be selected from
homopolymers and copolymers of N-vinyl lactams, especially homopolymers and co-polymers of N-vinyl pyrrolidone, e.g. polyvinylpyrrolidone (PVP), copolymers of N-vinyl pyrrolidone and vinyl acetate or vinyl propionate,
cellulose derivatives, such as cellulose esters, cellulose ethers and cellulose ether esters, in particular methylcellulose and ethylcellulose, hydroxyalkylcelluloses, in particular hydroxypropylcellulose, hydroxyalkylalkylcelluloses, in particular hydroxypropylmethylcellulose, cellulose phthalates or succinates, in particular cellulose acetate phthalate and hydroxypropylmethylcellulose phthalate, hydroxypropylmethylcellulose succinate or hydroxypropylmethylcellulose acetate succinate;
high molecular polyalkylene oxides such as polyethylene oxide and polypropylene oxide and copolymers of ethylene oxide and propylene oxide,
polyvinyl alcohol-polyethylene glycol-graft copolymers (available as Kollicoat® IR from BASF SE, Ludwigshafen, Germany);
graft copolymers comprising a poly(alkylene glycol) backbone and a vinyl acetate/N-vinylcaprolactam copolymer grafted onto the backbone (available as Soluplus® from BASF SE, Ludwigshafen, German);
polyacrylates and polymethacrylates such as methacrylic acid/ethyl acrylate copolymers, methacrylic acid/methyl methacrylate copolymers, butyl methacrylate/2-dimethyl-aminoethyl methacrylate copolymers, poly(hydroxyalkyl acrylates), poly(hydroxyalkyl methacrylates),
polyacrylamides,
vinyl acetate polymers such as copolymers of vinyl acetate and crotonic acid, partially hydrolyzed polyvinyl acetate (also referred to as partially saponified "polyvinyl alcohol"),
polyvinyl alcohol,
oligo- and polysaccharides such as carrageenans, galactomannans and xanthan gum,
polyhydroxyalkanoates, e.g. polyhydroxybutyric acid, polylactic acid, and poly(lactideco-glycolide);
polyamino acids, e.g. polylysine, polyasparagine,
proteins and polypeptides, such as gelatin
or mixtures of one or more thereof.

According to one aspect of the invention, the nutritionally acceptable thermoplastic polymer is selected from cellulose derivatives, especially hydroxyalkylcelluloses and hydroxyalkylalkylcelluloses.

Preferred water-soluble polymers are for example hydroxypropylmethylcellulose (Methocel, Pharmacoat), polymethacrylate (Eudragit EO), hydroxypropylcellulose (Klucel), or a polyvidone.

Especially preferred water-soluble polymers are hydroxypropylmethylcelluloses or HPMC (INN name: hypromellose). Said HPMC contains sufficient hydroxypropyl and methoxy groups to render it water-soluble. HPMC having a methoxy degree of substitution from about 0.8 to about 2.5 and a hydroxypropyl molar substitution from about 0.05 to about 3.0 are generally water-soluble. Methoxy degree of substitution refers to the average number of methyl ether groups present per anhydroglucose unit of the cellulose molecule. Hydroxypropyl molar substitution refers to the average number of moles of propylene oxide which have reacted with each anhydroglucose unit of the cellulose molecule. Preferably hydroxypropylmethylcellulose with low viscosity, i.e. about 5 mPa·s, is used, e.g. hydroxypropylmethylcellulose 2910 5 mPa·s. In the four digit number "2910", the first two digits represent the approximate percentage of methoxyl groups and the third and fourth digits the approximate percentage composition of hydroxypropoxyl groups. 5 mPa·s is a value indicative of the apparent viscosity of a 2 % aqueous solution at 20°C. Suitable HPMC include those having a viscosity from about 1 to about 100 mPa·s, in particular form about 3 to about 15 mPa·s, preferably about 5 mPa·s The most preferred type of HPMC having a viscosity of 5 mPa·s., is the commercially available HPMC 2910 5 mPa·s.

The term "phosphatide", as used herein, refers to compounds which are derivatives of glycero-3 phosporic acid that contains at least one O-acyl, O-alkyl or O-alk-1'-enyl residue attached to the glycerol moiety and a polar head made of a nitrogenous base, a glycerol, or an inositol unit. The terms "phosphatide", "glycerophospholipid" and "phosphoglyceride" are used interchangeably. In a further particular embodiment of the invention the phosphatide comprised in the solid dispersion product described herein is a lecithin. Lecithins are in particular phosphatidylcholines, i.e. a group of phosphatides composed of phosphoric acid, choline, and fatty acids.

According to one aspect of the invention, the solid dispersion product additionally comprises a normally solid polyol, i.e. "normally solid" means that the polyol is solid at NTP (Normal Temperature and Pressure, i.e., 20 °C and 1 atm). The normally solid polyol acts as a melting point or softening point depressant and facilitates the uniform incorporation of the curcuminoid(s) into the solid dispersion matrix. Without wishing to be bound to theory, we believe that the normally solid polyol acts as a plasticizer for the nutritionally acceptable polymer and/or the normally solid polyol initially melts and the other component(s) will dissolve in the melt.

The normally solid polyol can be selected from, e. g., high molecular weight polethylenglycols.

Preferably, however, said polyol is a sugar alcohol. A sugar alcohol is a hydrogenated form of carbohydrate, whose carbonyl group has been reduced to a primary or secondary hydroxyl group. Said sugar alcohol may be selected from maltitol, mannitol, sorbitol, lactitol, xylitol, erythritol and isomalt. In a particular embodiment of the invention the polyol is isomalt, also referred to as 1-O-alpha-D-glycopyranosyl-D-mannitol or Palatinit.

The solid dispersion product described herein may comprise
a) 0.1 to 50%, for example 5 to 30% or 10 to 20%, by weight of the curcuminoid(s),
b) 20 to 95%, for example 40 to 80%, by weight of the nutritionally acceptable thermoplastic polymer,
c) 5 to 50%, for example 5 to 25%, by weight of the phosphatide, and
d) 0 to 50%, for example 1 to 30% or 5 to 15%, by weight of the normally solid polyol.

Various additives may be included in the curcuminoid formulation of the invention, for example lubricants, fillers, disintegrants, preservatives or stabilizers such as antioxidants, light stabilizers, radical scavengers and stabilizers against microbial attack, dyes such as azo dyes, organic or inorganic pigments such as iron oxides or titanium dioxide, or dyes of natural origin, pH regulators, as well as compounds which alter or mask flavor and/or odor of the curcuminoid formulation such as sweeteners, flavorings and odorants.

At least part of the curcuminoid(s) is/are homogeneously dispersed in the matrix of the solid dispersion product. It is particulary preferred that the curcuminoid(s) in the solid dispersion product is/are present in an essentially non-crystalline state. This encompasses a state wherein essentially amorphous domains of curcuminoid(s) are interspersed in the matrix and a state wherein the curcuminoid(s) are molecularly dispersed in the matrix. When said dispersion of the components is such that the system is chemically and physically uniform or homogeneous throughout or consists of one phase (as defined in thermodynamics), such a solid dispersion will be called a "solid solution" or a "molecular dispersion". The state of molecular dispersion corresponds to the maximum possible homogenization of the curcuminoid in the matrix.

Known analytical methods can be used to investigate the state of such solid dispersions, for example differential scanning calorimetry (DSC) or wide angle X-ray scattering measurements (WAXS measurements). The DSC analytical measurement of an essentially non-crystalline state lacks the melting peak which occurs with the crystalline pure substance and is usually endothermic. Another possibility for identifying an essentially non-crystalline state is the reduction in intensity and/or absence of typical X-ray diffraction signals in the WAXS analysis.

Stability of curcuminoids is particularly high under dry conditions. Therefore the curcumin formulations of the invention preferably comprise less than 5% per weight, for example less that 1% per weight, water. In a particular embodiment of the invention the curcumin formulation is essentially anhydrous.

The solid dispersion product can be produced by blending one or more curcuminoids, a nutritionally acceptable thermoplastic polymer, and a phosphatide, heating the blend to obtain a homogeneous melt, and allowing the melt to solidify to obtain a solid dispersion product. The terms "melt" and "melting" should be interpreted broadly. For the purposes herein, these terms not only mean the alteration from a solid state to a liquid state, but can also refer to a transition to a glassy state or a rubbery state, and in which it is possible for one component of the mixture to get embedded more or less homogeneously into the other. In particular cases, one component will melt and the other component(s) will dissolve in the melt, thus forming a solution, which, upon cooling, may form a solid solution having advantageous dissolution properties. Blending and heating are conveniently performed in a mixer or kneader which is jacketed for heating.

A preferred method for producing the curcuminoid formulation of present invention comprises:
a) blending one or more curcuminoids, a nutritionally acceptable thermoplastic polymer, and a phosphatide;
b) heating the blend to obtain a homogeneous melt;
c) forcing the thus obtained melt through one or more nozzles;
d) allowing the melt to solidify to obtain a solid dispersion product.

Steps a) to c) may be performed in one or more than one apparatus suitable for this purpose, such as an extruder or kneader extruder. Preferable the blend is subjected to a mixing action in a mixing section of the extruder.

Extruders are known per se. An extruder comprises a housing or barrel divided into several sections in a longitudinal direction. On the upstream side of the extruder, an opening is provided for feeding powders of the curcuminoid(s), the nutritionally acceptable thermoplastic polymer, the phosphatide and any further components such as the normally solid polyol and/or additives described above. Usually, a hopper is placed on this opening so that the powder can be easily fed into the barrel of the extruder. The barrel ends in conveying direction in a die, where the dispersion is expelled.

The extruder comprises at least one rotating shaft. Alternatively, it may comprise two or up to six rotating shafts. In preferred embodiments, the extruder is a twin-screw extruder. The shafts may be co-rotating or counter-rotating, but are preferably co-rotating. Processing elements disposed on adjacent shafts closely intermesh.

Each shaft carries a plurality of processing elements disposed axially one behind the other. The processing elements define a feeding and conveying section, at least one mixing section, and a discharging section. The feeding and conveying section is positioned farthest upstream, close to the hopper of the extruder, the at least one mixing section is positioned downstream of the feeding and conveying section, and the discharging section is positioned farthest downstream, close to the discharge opening of the extruder. The term "downstream" as used herein, refers to direction in which the material is being conveyed in the extruder, i.e. the conveying direction.

The processing elements of the feeding and conveying section as well as the discharging section are formed by screw-type elements. Preferably, these screw type elements form an endless screw having the feed direction and a uniform pitch flight. Thus, in the feeding and conveying section the powder is fed into the extruder and conveyed in the downstream direction, for example at a feed rate of 0.5 to 1.5 h, preferably of 0.5 to 1.0 kg/h.

In the mixing section(s) the material to be processed is homogenized by mixing or kneading. Suitably, paddle means or kneading blocks may be used. These kneading blocks consist of cam disks mutually offset at an angle in a peripheral direction. The cam disks have abutting faces that are perpendicular to the general conveying direction in the extruder. Alternatively, the mixing section(s) are defined by processing element(s) that comprise(s) a mixing element that is derived from a screw type element. A mixing element "being derived from a screw type element" is intended to mean an element whose basic shape is that of a screw element, but which has been modified such that it exerts a compounding or mixing effect in addition to a conveying effect.

In a preferred embodiment of the invention, the extruder comprises one or more than one, for example three or four, mixing sections, which are connected by intermediate conveying sections formed by screw-type elements.

The extruder shaft may further comprise one or more than one reverse-flight section(s), preferably arranged after the (last) mixing section and defined by reverse-flight elements. A reverse-flight element has a screw with a reverse-flight relative to the screw-type elements which may be arranged in the feeding and conveying section which define the general conveying direction of the extruder. Thus, the reverse-flight element convey the material in an opposite direction relative to the general conveying direction of the extruder and serves to create sufficient back-pressure to allow for a desired degree of mixing and/or homogenization. The reverse-flight element is designed to stow the material conveyed in the extruder. Therefore it may also be called a back-pressure element.

The substances which are fed to the extruder are melted in order to homogenize the melt and to disperse or, preferably, dissolve the curcuminoid in the matrix efficiently. "Melting" means transition into a liquid or rubbery state in which it is possible for one component to be homogeneously embedded in the other. Melting usually involves heating above the softening point of the polymer. Usually, the maximum melt temperature is in the range of 90 to 180°C, preferably 100 to 160°C.

The extruder housing is heated in order to form a melt from the substances fed to the extruder. It will be appreciated that the working temperatures will also be determined by the kind of extruder or the kind of configuration within the extruder that is used. A part of the energy needed to melt, mix and dissolve the components in the extruder can be provided by heating elements, while the friction and shearing of the material in the extruder can also provide the mixture with a substantial amount of energy and aid in the formation of a homogeneous melt of the components.

In order to obtain a homogeneous distribution and a sufficient degree of dispersion of the active ingredient, the curcuminoid-containing melt is kept in the heated barrel of the melt extruder for a sufficient length of time.

According to one aspect of the invention, the barrel of the extruder is divided into several heating zones. The temperature in these heating zones can be controlled in order to control the melting of the dispersion. Preferably, the portion of the barrel upstream of the first mixing element or first reverse-flight element is maintained at a lower temperature, e.g. a temperature of about 50 to about 100°C, than the portion of the barrel downstream of the first mixing element that is kept at, e.g., about 100 to about 150°C.

The extrudate exiting from the extruder ranges from pasty to viscous. Before allowing the extrudate to solidify, the extrudate may be directly shaped into virtually any desired shape. Shaping of the extrudate may be conveniently carried out by a calender with two counter-rotating rollers with mutually matching depressions on their surface. A broad range of tablet forms can be attained by using rollers with different forms of depressions. If the rollers do not have depressions on their surface, films can be obtained. Alternatively, the extrudate is molded into the desired shape by injection-moulding. Alternatively, the extrudate is subjected to profile extrusion and cut into pieces, either before (hot-cut) or after solidification (cold-cut).

Optionally, the resulting solid dispersion product is milled or ground to granules. The granules may then be compacted. Compacting means a process whereby a powder mass comprising the granules is condensed under high pressure in order to obtain a compact with low porosity, e.g. a tablet. Compression of the powder mass is usually done in a tablet press, more specifically in a steel die between two moving punches.

A number of uses may be contemplated for the curcuminoid formulations of the present invention. In particular, the curcumin formulations of the invention can be used in the nutritional and pharmaceutical sector for both, humans and non-human animals. For example, said applications comprise the use of the curcumin formulations in the treatment or prophylaxis of diseases and conditions including cancer, in particular solid tumors such as colorectal, lung, breast, pancreatic and prostate carcinoma. Further potential pharmacological uses are in the treatment or prophylaxis of conditions involving an inflammatory reaction such as arthritis, neurological disorders such as Alzheimer's disease and Parkinson's disease, cardiovascular disease, pulmonary disease, the formation of cholesterol gallstones, and parasitic infestation such as by *Plasmodium* (e.g. malaria pathogen), *Trypanosoma* and *Leishmania.* For these purposes, the formulations of the invention may be administered alone, for example as dietary supplements, or together with further excipients, drugs or as food ingredients.

For oral administration of the formulations of the invention a variety of dosage forms may be used comprising liquid or semisolid forms such as emulsions, microemulsions and suspensions, and solid forms such as granules, capsules, pellets, powders or tablets.

Emulsions and microemulsions may be of the oil-in-water or water-in-oil type and contain formulations of the invention as disperse or dispersing phase. These emulsions or microemulsions may be stabilized by the presence of emulsifiers known to be used for this purpose.

Granules consist of solid grains of formulations of the invention, each grain representing an agglomerate of powder particles. A lubricant is preferably used in compacting the granules. Suitable lubricants are selected from polyethylene glycol (e.g., having an Mw of from 1000 to 6000), magnesium and calcium stearates, sodium stearyl fumarate, and the like. The user can be offered single-dose preparations, for example granules packed in a small bag (sachet), a paper bag or a small bottle, or multidose preparations which require appropriate dimensions. However, in many cases, such granules do not represent the actual drug form, but are intermediates in the manufacture of particular drug forms, for example tablet granules to be compressed to tablets, capsule granules to be packed into hard gelatin capsules, or instant granules or granules for oral suspension to be put in water before intake.

As capsules, the formulations of the invention are usually packed into a hard shell composed of two pieces fitted together or a soft, one-piece, closed shell, which may vary in shape and size. It is likewise possible for formulations of the invention to be encased or enveloped or embedded in a matrix in suitable polymers, i.e. microcapsules and microspherules. Hard and soft capsules consist mainly of gelatin, while the latter have a suitable content of plasticizing substances such as glycerol or sorbitol. Hard gelatin capsules are used to receive formulations of the invention which have a solid consistency, for example granules, powder or pellets. Soft gelatin capsules are particularly suitable for formulations with a semisolid consistency and, if required, also viscous liquid consistency.

Pellets are granules of formulations of the invention in the particle size range from about 0.5 to 2 mm in diameter. Both with a narrow particle size distribution, preferably from 0.8 to 1.2 mm, and with an essentially round shape, are preferred.

Tablets are solid preparations in particular for oral use. The meaning of "oral administration" within the framework of the present invention is, in particular, that of the term "peroral administration" or "ingestion", thus the tablets are for absorption or action of the curcuminoid in the gastrointestinal tract. Particular embodiments are coated tablets, layered tablets, laminated tablets, tablets with modified release of the curcuminoid, matrix tablets, effervescent tablets, chewable tablets or pills. The formulations of the invention usually comprise at least a part of the necessary tablet excipients, such as binders, fillers, glidants and lubricants, and disintegrants. Tablets of formulations of the invention may also, if necessary, comprise other suitable excipients, for example excipients which assist tableting such as lubricants and glidants, e.g. magnesium, aluminum and calcium stearates, talc and silicones, animal or vegetable fats, especially in hydrogenated form and those which are solid at room temperature. Coated tablets additionally comprise suitable coating materials, for example film coating agents with coating aids, especially those mentioned below. Coated tablets include, in particular, sugar-coated tablets and film-coated tablets.

Powders are finely dispersed solids of formulations of the invention with particle sizes usually of less than 1 mm. The above statements about granules apply correspondingly.

The curcuminoid formulations of the present invention show improved oral bioavailability compared to non-formulated crystalline curcuminoid(s). The oral bioavailability can be determined in experiments involving oral administration of the curcuminoid formulation of the invention (or an corresponding amount of non-formulated curcuminoid(s)) to a subject and measuring the level of the curcuminoid(s) in a biological sample obtained from the subject over time, wherein the biological sample may be derived from a body fluid; for example serum, plasma, whole blood, or cerebrospinal fluid, and/or a tissue, e.g. from brain, liver, kidney or heart. For analysis, the curcuminoid level in the examined body fluid or tissue may be plotted against time, and the area under the curve (AUC), for example the total area under the curve from t = 0 (time of administration) to t = ∞ (= AUC_{0-∞}), or the area under the curve within a defined period, e.g. from t = 0 to t = 6 h (AUC₀₋₆ₕ), may be calculated. In general, a higher AUC relative to the AUC obtained by administration of non-formulated crystalline curcuminoid(s) indicates an improved bioavailability. The absolute bioavailability may be calculated from the resulting AUC data as percentage based on the corresponding AUC data obtained from intravenous administration of curcuminoid(s).

In particular aspects of the invention, the amount of curcuminoid in the blood, determined as AUC₀₋₆ₕ, after a single oral administration of a dose of the curcuminoid formulation of the present invention corresponding to 20 mg of total curcuminoids to a human subject or an animal subject, preferably to a rat, is significantly higher than after oral administration of the same amount of non-formulated crystalline curcuminoids, preferably at least 2 times, at least 3 times, at least 4 times, at least 6 times, at least 8 times, at least 10 times, or at least 15 times, and, for example, up to 30 times higher.

As used herein, the amount of curcuminoid in the blood being "significantly higher" means a statistically significant increase of this parameter in subjects after oral administration of 20 mg curcuminoid comprised in the formulation of the invention compared to the control (20 mg non-formulated crystalline curcuminoids). A statistical test known in the art, such as ANOVA or Student's t-test, may be used to determine the significance of this difference, wherein the p-value is at least <0.1, <0.05, <0.01, <0.005, <0.001 or <0.0001.

### EXAMPLES

### EXAMPLE 1: Preparation and analysis of solid dispersion products for curcuminoid formulations

Apparatus:
twin-screw extruder ZSK 18 MEGAlab (Coperion) equipped with a gravimetric feeder and a strand die with a diameter of 3 mm
Screw configurations A and B as described below

For the experiment described below two alternative types of extruder shafts with different screw configurations were applied. Each type of shaft carried a number of processing elements disposed axially one behind the other over the total shaft length arranged in three sections. About on third of the shaft positioned farthest upstream was a feeding and conveying section followed by about a further third comprising several mixing sections connected by intermediate conveying sections. The mixing sections comprised kneading blocks which consisted of cam disks mutually offset at an angle in a peripheral direction. The about one third of the shaft positioned farthest downstream was a discharging section.

Screw configuration A comprised four mixing sections. Screw configuration B comprised three mixing sections and was more aggressive than screw configuration A due to the presence of back-pressure elements designed to intensify mixing and/or homogenization.

The extruder barrel was divided into five heating zones. Heating zones 1 and 2 covered the feeding and conveying section. The mixing sections and the discharging sections were located in heating zones 3 to 5.

Materials:
hydroxpropylmethylcellulose (HPMC 2910 5 mPa·s)
lecithin (Ultralec® F Lecithin)
isomalt (Isomalt PF)
curcuminoids (Curcumin C3 Complex, Sabinsa)

### Procedure:

Four different powder premixes, #1, #2, #3 and #4, 1.5 kg each, were prepared by manually blending the ingredients shown in Table 1 in a PE bag. Samples of the premixes were fed separately to the twin-screw extruder. Melt extrusion was performed under the conditions summarized in Table 2. The samples were tested on residual crystalline curcuminoids after extrusion (Table 3). No crystallinity was detectable in the solid dispersion products resulting from processing of samples #1.1, #1.2, #2.1 and #2.2 having a curcuminoid load of 10% w/w. The solid dispersion products of sample #3.1-3 and #4.1-3 having a curcuminoid load of 20% w/w showed various levels of residual curcuminoid crystallinity related to and consistent with the applied process parameters.

**TABLE 1: Composition of premixes**

| Premix | Raw material | Amount [% w/w] | Amount [g] |
|---|---|---|---|
| #1 | HPMC | 60 | 900 |
| | Isomalt | 10 | 150 |
| | Lecithin | 20 | 300 |
| | Curcuminoids | 10 | 150 |
| #2 | HPMC | 75 | 1125 |
| | Isomalt | 5 | 75 |
| | Lecithin | 10 | 150 |
| | Curcuminoids | 10 | 150 |
| #3 | HPMC | 50 | 750 |
| | Isomalt | 10 | 150 |
| | Lecithin | 20 | 300 |
| | Curcuminoids | 20 | 300 |
| #4 | HPMC | 65 | 975 |
| | Isomalt | 5 | 75 |
| | Lecithin | 10 | 150 |
| | Curcuminoids | 20 | 300 |

**TABLE 2: Parameters used for melt extrusion**

| Sample | Feed rate [kg/h] | Vacuum [mbar] | Screw speed [rpm] | Torque [%] | Temperature profil of heating zones 1-5 [°C] | Melt temp. at die [°C] | Screw configuration |
|---|---|---|---|---|---|---|---|
| #1.1 | 0.75 | non | 200 | 12 | 50/100/130/140/140 | 142 | A |
| #1.2 | 0.75 | 500 | 150 | 11-12 | 50/100/130/140/140 | 144 | A |
| #2.1 | 0.75 | non | 200 | 14-15 | 50/100/130/140/150 | 155 | A |
| #2.2 | 0.75 | non | 150 | 33 | 50/100/130/140/150 | 158 | B |
| #3.1 | 0.75 | 540 | 150 | 21 | 50/100/120/130/140 | 145 | B |
| #3.2 | 1.1 | 540 | 150 | 30 | 50/100/120/130/140 | 144 | B |
| #3.3 | 0.75 | 560 | 125 | 29 | 50/90/110/120/130 | 134 | B |
| #4.1 | 0.75 | non | 125 | 32 | 50/90/110/120/130 | 137-139 | B |
| #4.2 | 0.75 | non | 124 | 52 | 50/90/100/110/120 | 122 | B |

**TABLE 3: Product after melt extrusion**

| Sample | Strand color | Comment | Curcuminoid crystallinity (mean of n=2)* |
|---|---|---|---|
| #1.1 | brown | yellow | below detection limit |
| # 1.2 | lighter brown | agglomerates, more briddle | below detection limit |
| #2.1 | brown | good homogentity | below detection limit |
| #2.2 | dark brown | yellow agglomerates | below detection limit |
| #3.1 | brown | yellow agglomerates | ~ 11% |
| #3.2 | yellow/brown | less agglomerates | ~ 20% |
| #4.1 | light brown | smooth, homogen | ~ 11% |
| #4.2 | yellow/orange | smooth, homogen | ~ 28% |

| | | | |
|---|---|---|---|
| * % curcuminoid crystallinity based on the total amount of curcuminoid | | | |

### EXAMPLE 2: Bioavailability of orally administered curcuminoid formulations in rats Animals:

- adult Sprague Dawley rats (14-16 weeks old, average body weight 260-280 g) Animals were obtained from Harlan Laboratories, Netherlands. Rats were acclimatized to the study area conditions for 3 days before dosing. Animals were housed in polycarbonate cages and maintained in controlled environmental conditions with 12 hr light and dark cycles. The temperature and humidity of the room ranged between 21 to 24°C and 50 to 70%, respectively. Animals were fed rat pellet food *ad libitum* except when fasted and were provided with fresh water. The animals were fasted for 12 h prior to dose administration.

### Study design:

Each dosing group consisted of 5 rats. Rats were implanted with cannula in the jugular vein for blood sampling. The surgical preparation was performed under anesthesia two days before dosing as per approved protocols. In a typical study, animals were orally dosed with test substance. Blood samples were taken prior to dosing and 15, 30, 60, 90, 120, 240 and 360 min after dosing. K2-EDTA was used as the anti-coagulant. Blood samples were collected from the jugular vein of cannulated rats in all cases. Plasma was collected by centrifugation and stored at -80°C until bioanalysis. Quantitative bioanalysis of formulated curcuminoids in the plasma samples were processed using protein precipitation with acetonitrile and analyzed by LC-MS/MS.

### Test samples:

- curcuminoid formulations: samples #1.1, #1.2,. #2.1, #2.2, #3.1, #3.2, #3.3, #4.1 and #4.2, prepared as described in example 1, stored at 4°C until use
- control: non-formulated crystalline curcuminoids

### Dosing of test samples:

On the day of dosing, dosing solutions were prepared by mixing the respective test sample with 0.25% carboxymethylcellulose in H₂O. Each animal was orally administered witch ∼2.5 ml dosing solution comprising 20 mg curcuminoids:

### Determination of curcuminoids in rat plasma:

0.1 ml plasma was precipitated using 0.25 ml of acetonitrile containing warfarin (1000 µg/ml) as an internal standard. Precipitated protein was removed by centrifugation and supernatant fraction was analyzed by LC-MS/MS. Details of bioanalysis are given below:
- Curcumin LLOQ: 30:23.ng/ml ULOQ:3415 ng/ml
- Desmethoxycurcumin LLOQ: 9.339 ng/ml ULOQ: 1055 ng/ml
- Bis-desmethoxycurcumin LLOQ: 1.676 ng/ml ULOQ: 189.0 ng/ml
- r² value: plasma: -0.9800
- Instrumentation: API 4000 LC-MS/MS with 1.4.2 Analyst version
- Mode: ESI Negative mode
- Experiment Type: MRM
- Ion source type & ionization mode: ESI

**TABLE 4: Transition details in LC-MS/MS**

| Analyte | MS/MS transition | Dwell (msec) | DP (Volts) | EP (Volts) | CE (Volts) | CXP (volts) |
|---|---|---|---|---|---|---|
| Curcumin | 367/133.90 | 150 | -63 | 10 | -43 | -7 |
| Desmethoxy curcumin | 306.9/118.90 | 150 | -60 | 10 | -24 | -7 |
| Bis-desmethoxy curcumin | 306.9/118.9 | 150 | -60 | 10 | -24 | -7 |
| Curcumin glucuronide | 543. /134.0 | 150 | -63 | 10 | -43 | -7 |
| Desmethoxy glucuronide | 513.0/118:9 | 150 | -58 | 10 | -24 | -7 |
| Bisdesmethoxy glucuronide | 483.0/118.9 | 150 | -60 | 10 | -24 | -7 |
| ISTD Warfarin | 307.2/161.2 | 150 | -85 | 10 | -43 | -9 |

| Interface heater | Ion source (GS1) | CAD | CUR | TEM | IS | GS2 |
|---|---|---|---|---|---|---|
| ON | 5 | 6 | 10 | 450 | 5500 | 45 |

| | | | | | | |
|---|---|---|---|---|---|---|
| HPLC conditions • HPLC type: Shimadzu UFLC (XR) • Column: Agilent Eclipse XDB C18; 100 x 4.6 mm; 5 µ. • Injection volume: 15 µl • Auto sampler & Column Temperature: Auto sampler 5°C; Column temperature 40°C • Mobile phase A: 0.1% formic acid • Mobile phase B: 0.1% formic acid in acetonitrile • Flow rate 1.0 ml/min with split at 80:20 (20% into mass) • Total sample analysis time: 10.0 min | | | | | | |

### Determination of curcuminoid glucuronide concentration in plasma:

In this study, it was observed that curcuminoids were rapidly converted to conjugates of glucuronide in rat plasma. Since reference standards for curcuminoid glucuronides were not available, these conjugates were generated *in vitro* using rat liver S9 fraction and further purified. Curcuminoid glucuronides were quantified using the reported molecular extinction coefficient for curcumin (4.88 x 10⁴ M⁻¹ cm⁻¹ at 420 nm). (Our finding that purified curcumin glucuronide showed absorption maxima at 254 nm, similar to curcuminoids strongly suggests that glucuronide conjugation does not affect the spectral properties of the chromophore in the curcumin molecule.) Since the response in LC-MS/MS for curcumin and its glucuronide conjugate were not similar, a correction factor of 60 was applied to obtain approximate levels of curcuminoid glucuronides formed in rat plasma. (Said correction factor was determined as the ratio of the peak area counts obtained for similar amounts (∼1.4 ng) of curcumin (peak area count: 613007) and its glucuronide conjugate (peak area count: 10103).)

### Data analysis and determination of PK parameters:

Data obtained from bioanalysis was analyzed using WinNonlin (V5.2.1) to determine various PK parameters such as Kₑₗ, t_{1/2}, Tₘₐₓ, Cₘₐₓ, AUC₀₋₆ₕ, and AUC_{0-∞}.

### Results:

Calculated levels of curcuminoids (total curcuminoid glucuronides) in rat plasma measured from 0 min up to 360 min after administration of dosing solution and the respective pharmacokinetic parameters are shown in Fig.1 A-J and Table 5.

**TABLE 5: Pharmacokinetic parameters for oral administration of curcuminoid formulations to rats**

| Test sample / no. of rats | | Kel[1/h] | T_{1/2} [h] | Tₘₐₓ [h] | Cₘₐₓ [ng/ml] | AUC₀₋₆ₕ [h·ng/ml] | AUC_{0-∞} [h·g/ml] |
|---|---|---|---|---|---|---|---|
| #1.1 / 5 | Mean | 0.77 | 0.90 | 1.50 | 3947 | 7401 | 7493 |
| | STDEV¹⁾ | 0.04 | 0.05 | 0.35 | 737 | 585 | 587 |
| #1.2/4²⁾ | Mean | 0.74 | 0.95 | 0.88 | 2945 | 4706 | 4751 |
| | STDEV | 0.10 | 0.13 | 0.22 | 310 | 384 | 381 |
| #2.1/5 | mean | 0.72 | 0.97 | 2.00 | 3743 | 7357 | 7547 |
| | STDEV | 0.08 | 0.11 | 0.00 | 523 | 1635 | 1671 |
| #2.2 / 5 | Mean | 0.82 | 0.85 | 1.10 | 3795 | 6639 | 6702 |
| | STDEV | 0.07 | 0.08 | 0.55 | 633 | 637 | 642 |
| #3.1 3²⁾ | Mean | 0.64 | 1.24 | 2.00 | 1593 | 3501 | 3738 |
| | STDEV | 0.24 | 0.60 | 0.00 | 179 | 549 | 361 |
| #3.2/4²⁾ | Mean | 1.08 | 0.67 | 0.50 | 1439 | 1939 | 1945 |
| | STDEV | 0.26 | 0.19 | 0.35 | 406 | 851 | 850 |
| #3.3 / 5 | Mean | 0.46 | 1.66 | 1.00 | 809 | 1455 | 1597 |
| | STDEV | 0.15 | 0.61 | 0.61 | 332 | 339 | 373 |
| #4.1 / 5 | mean | 1.20 | 0.63 | 0.35 | 1377 | 2149 | 2155 |
| | STDEV | 0.38 | 0.20 | 0.14 | 617 | 720 | 723 |
| #4.2 / 4²⁾ | Mean | 0.68 | 1.05 | 1.38 | 1624 | 3403 | 3502 |
| | STDEV | 0.11 | 0.18 | 0.25 | 355 | 531 | 521 |
| control / 5 | Mean | 1.06 | 0.67 | 1.00 | 174 | 377 | 419 |
| | STDEV | 0.19 | 0.12 | 0.00 | 57 | 114 | 109 |

| | | | | | | | |
|---|---|---|---|---|---|---|---|
| ¹⁾ STDEV = standard deviation ²⁾ #1.2/animal-3, #3.1/animal-8, #3.2/animal-4, and #4.2/animal-5 were excluded from calculation of means and standard deviations. ' | | | | | | | |

## Claims

1. A curcuminoid formulation, comprising a melt-processed solid dispersion product comprising
a) one or more curcuminoids,
b) a nutritionally acceptable thermoplastic polymer, and
c) a phosphatide.

2. The curcuminoid formulation of claim 1, wherein the solid dispersion product additionally comprises a normally solid polyol.

3. The curcuminoid formulation of claim 1 or 2, wherein the cureuminoid(s) are present in an essentially non-crystalline state.

4. The curcuminoid formulation of any of claims 1 to 3, wherein the melt-processed solid dispersion product comprises
a) 0.1 to 50% by weight of the curcuminoid(s),
b) 20 to 95% by weight of the nutritionally acceptable thermoplastic polymer,
c) 5 to 50% by weight of the phosphatide, and
d) 0 to 50% by weight of the normally solid polyol.

5. The curcuminoid formulation of any of claims 1 to 4, wherein the nutritionally acceptable thermoplastic polymer is selected from the group consisting of homopolymers and copolymers of N-vinyl lactams, cellulose derivatives, high molecular polyalkylene oxides, polyvinyl alcohol-polyethylene glycol-graft copolymers, graft copolymers comprising a poly(alkylene glycol) backbone and a vinyl acetate/N-vinylcaprolactam copolymer grafted onto the backbone, polyacrylates and polymethacrylates, polyacrylamides, vinyl acetate polymers, polyvinyl alcohol, oligo- and polysaccharides, polyhydroxyalkanoates, polyamino acids, proteins and polypeptides and mixtures thereof.

6. The curcuminoid formulation of claim 5, wherein the nutritionally acceptable thermoplastic polymer is selected from cellulose derivatives.

7. The curcuminoid formulation of claim 6, wherein the nutritionally acceptable thermoplastic polymer is hydroxypropyl methyl cellulose.

8. The curcuminoid formulation of any of claims 2 to 7, wherein the normally solid polyol is a sugar alcohol.

9. The curcuminoid formulation of claim 8, wherein the normally solid polyol is isomalt.

10. The curcuminoid formulation of any of claims 1 to 9, wherein the phosphatide is lecithin.

11. The curcuminoid formulation of any one of claims 1 to 10, wherein the amount of curcuminoid in the blood of a rat subject determined as AUC₀₋₆ₕ after a single oral administration of a dose of the formulation corresponding to 20 mg of curcuminoid to said subject is at least 2 times higher than after oral administration of the same amount of non-formulated crystalline curcuminoid.

12. A nutritional product fortified with a curcuminoid formulation of any of claims 1 to 11.

13. The curcuminoid formulation of any of claims 1 to 11 for use in the treatment or prophylaxis of cancer, conditions involving an inflammatory reaction, neurological disorders, cardiovascular disease, pulmonary disease, the formation of cholesterol gallstories, and parasitic infestation.

14. A method for producing the curcuminoid formulation of any of claims 1 to 11, comprising:
a) blending one or more curcuminoids, a nutritionally acceptable thermoplastic polymer, and a phosphatide;
b) heating the blend to obtain a homogeneous melt;
c) forcing the thus obtained melt through one or more nozzles;
d) allowing the melt to solidify to obtain a solid dispersion product.

15. The method of claim 14, wherein step b) is carried out in an extruder and the blend is subjected to a mixing action in a mixing section of the extruder.

## Patentansprüche

1. Eine Curcuminoid-Formulierung, aufweisend ein schmelzverarbeitetes festes Dispersionsprodukt, aufweisend
a) ein oder mehrere Curcuminoide,
b) ein ernährungsbezogen akzeptables thermoplastisches Polymer, und
c) ein Phosphatid.

2. Die Curcuminoid-Formulierung von Anspruch 1, wobei das feste Dispersionsprodukt zusätzlich ein normalerweise festes Polyol aufweist.

3. Die Curcuminoid-Formulierung von Anspruch 1 oder 2, wobei das Curcuminoid / die Curcuminoide in einem im Wesentlichen nicht-kristallinen Zustand vorhanden ist / sind.

4. Die Curcuminoid-Formulierung von irgendeinem der Ansprüche 1 bis 3, wobei das schmelzverarbeitete feste Dispersionsprodukt aufweist
a) 0,1 bis 50 Gewichts-% des Curcuminoids / der Curcuminoide,
b) 20 bis 95 Gewichts-% des ernährungsbezogen akzeptablen thermoplastischen Polymers,
c) 5 bis 50 Gewichts-% des Phosphatids, und
d) 0 bis 50 Gewichts-% des normalerweise festen Polyols.

5. Die Curcuminoid-Formulierung von irgendeinem der Ansprüche 1 bis 4, wobei das ernährungsbezogen akzeptable thermoplastische Polymer ausgewählt ist aus der Gruppe bestehend aus Homopolymeren und Copolymeren von N-Vinyllactamen, Cellulosederivaten, hochmolekularen Polyalkylenoxiden, Polyvinylalkohol-Polyethylenglycol-Pfropf-Copolymeren, Pfropf-Copolymeren, aufweisend ein Poly(alkylenglykol)-Rückgrat und ein Vinylacetat/N-Vinylcaprolactam-Copolymer, das auf das Rückgrat gepfropft ist, Polyacrylaten und Polymethacrylaten, Polyacrylamiden, Vinylacetat-Polymeren, Polyvinylalkohol, Oligo- und Polysacchariden, Polyhydroxyalkanoaten, Polyaminosäuren, Proteinen und Polypeptiden, und Mischungen davon.

6. Die Curcuminoid-Formulierung von Anspruch 5, wobei das ernährungsbezogen akzeptable thermoplastische Polymer von Cellulosederivaten ausgewählt ist.

7. Die Curcuminoid-Formulierung von Anspruch 6, wobei das ernährungsbezogen akzeptable thermoplastische Polymer Hydroxypropylmethylcellulose ist.

8. Die Curcuminoid Formulierung von irgendeinem der Ansprüche 2 bis 7, wobei das normalerweise feste Polyol ein Zuckeralkohol ist.

9. Die Curcuminoid-Formulierung von Anspruch 8, wobei das normalerweise feste Polyol Isomalt ist.

10. Die Curcuminoid-Formulierung von irgendeinem der Ansprüche 1 bis 9, wobei das Phosphatid Lecithin ist.

11. Die Curcuminoid-Formulierung von irgendeinem der Ansprüche 1 bis 10, wobei die Menge an Curcuminoid in dem Blut eines Ratten-Subjekts, die als AUC_{O}-₆ₕ nach einer einzelnen oralen Verabreichung einer Dosis der Formulierung, die 20 mg an Curcuminoid entspricht, an das besagte Subjekt bestimmt wird, mindestens 2 mal höher ist als nach oraler Verabreichung der gleichen Menge an nicht-formuliertem kristallinen Curcuminoid.

12. Ein ernährungsbezogenes Produkt, das mit einer Curcuminoid-Formulierung von irgendeinem der Ansprüche 1 bis 11 verstärkt ist.

13. Die Curcuminoid-Formulierung von irgendeinem der Ansprüche 1 bis 11 zur Verwendung bei der Behandlung oder Prophylaxe von Krebs, Zuständen, die eine entzündliche Reaktion involvieren, neurologischen Störungen, Herz-Kreislauf-Erkrankungen, Lungenerkrankungen, der Bildung von Cholesteringallensteinen und parasitärem Befall.

14. Ein Verfahren zum Herstellen der Curcuminoid-Formulierung von irgendeinem der Ansprüche 1 bis 11, aufweisend:
a) Mischen eines oder mehrerer Curcuminoide, eines ernährungsbezogen akzeptablen thermoplastischen Polymers, und eines Phosphatids;
b) Erhitzen des Gemisches, um eine homogene Schmelze zu erhalten;
c) Zwingen der dadurch erhaltenen Schmelze durch eine oder mehrere Extrudermündungen (*nozzles*);
d) Ermöglichen der Schmelze zu verfestigen, um ein festes Dispersionsprodukt zu erhalten.

15. Das Verfahren von Anspruch 14, wobei Schritt b) in einem Extruder durchgeführt wird und das Gemisch einem Mischvorgang in einem Mischabschnitt des Extruders unterzogen wird.

## Revendications

1. Formulation de curcuminoïde, comprenant un produit de dispersion solide traité à l'état fondu comprenant
a) un ou plusieurs curcuminoides,
b) un polymère thermoplastique nutritionnellement acceptable, et
c) un phosphatide.

2. Formulation de curcuminoïde selon la revendication 1, dans laquelle le produit de dispersion solide comprend de plus un polyol normalement solide.

3. Formulation de curcuminoïde selon la revendication 1 ou 2, dans laquelle le(les) curcuminoïde(s) sont présents dans un état essentiellement non cristallin.

4. Formulation de curcuminoïde selon l'une quelconque des revendications 1 à 3, dans laquelle le produit de dispersion solide traité à l'état fondu comprend
a) de 0,1 à 50 % en masse du(des) curcuminoïde(s),
b) de 20 à 95 % en masse du polymère thermoplastique nutritionnellement acceptable,
c) de 5 à 50 % en masse du phosphatide, et
d) de 0 à 50 % en masse du polyol normalement solide.

5. Formulation de curcuminoïde selon l'une quelconque des revendications 1 à 4, dans laquelle le polymère thermoplastique nutritionnellement acceptable est choisi dans le groupe constitué d'homopolymères et de copolymères de N-vinyllactames, de dérivés de cellulose, de poly(oxydes d'alkylène) de masse moléculaire élevée, de poly(alcool vinylique)-polyéthylène glycol-copolymères greffés, de copolymères greffés comprenant un squelette poly(alkylène glycol) et d'un copolymère d'acétate de vinyle/N-vinylcaprolactame greffé sur le squelette, de polyacrylates et de polyméthacrylates, de polyacrylamides, de polymères d'acétate de vinyle, de poly(alcool vinylique), d'oligo- et de polysaccharides, de polyhydroxyalcanoates, de poly(amino-acides), de protéines et de polypeptides et de mélanges de ceux-ci.

6. Formulation de curcuminoïde selon la revendication 5, dans laquelle le polymère thermoplastique nutritionnellement acceptable est choisi parmi des dérivés de cellulose.

7. Formulation de curcuminoïde selon la revendication 6, dans laquelle le polymère thermoplastique nutritionnellement acceptable est l'hydroxypropylméthylcellulose.

8. Formulation de curcuminoïde selon l'une quelconque des revendications 2 à 7, dans laquelle le polyol normalement solide est un alcool de sucre.

9. Formulation de curcuminoïde selon la revendication 8, dans laquelle le polyol normalement solide est l'isomalt.

10. Formulation de curcuminoïde selon l'une quelconque des revendications 1 à 9, dans laquelle le phosphatide est la lécithine.

11. Formulation de curcuminoïde selon l'une quelconque des revendications 1 à 10, dans laquelle la quantité de curcuminoïde dans le sang chez un sujet rat déterminée par AUC₀₋₆ₕ après une seule administration orale d'une dose de la formulation correspondant à 20 mg de curcuminoïde audit sujet est au moins 2 fois plus élevée qu'après une administration orale de la même quantité de curcuminoïde cristallin non formulé.

12. Produit nutritionnel fortifié avec une formulation de curcuminoïde selon l'une quelconque des revendications 1 à 11.

13. Formulation de curcuminoïde selon l'une quelconque des revendications 1 à 11 pour une utilisation dans le traitement ou la prophylaxie du cancer, d'états impliquant une réaction inflammatoire, de troubles neurologiques, d'une maladie cardiovasculaire, d'une maladie pulmonaire, de la formation de calculs biliaires de cholestérol, et d'une infestation parasitaire.

14. Procédé de production de la formulation de curcuminoïde selon l'une quelconque des revendications 1 à 11, comprenant :
a) la combinaison d'un ou plusieurs curcuminoïdes, d'un polymère thermoplastique nutritionnellement acceptable, et d'un phosphatide ;
b) le chauffage de la combinaison pour obtenir une masse en fusion homogène ;
c) l'introduction forcée de la masse en fusion ainsi obtenue à travers une ou plusieurs buses ;
d) la solidification de la masse en fusion pour obtenir un produit de dispersion solide.

15. Procédé selon la revendication 14, dans lequel l'étape b) est réalisée dans une extrudeuse et la combinaison est soumise à une action de mélange dans une section de mélange de l'extrudeuse.
